# EUROPEAN PATENT SPECIFICATION

(11) **EP 3 694 400 B1**
(45) Date of publication and mention of the grant of the patent: **18.11.2026**
(21) Application number: 18865751.4
(22) Date of filing: 15.10.2018
(51) Int. Cl.: A61B 5/00, A61B 5/087, A61B 5/113, G16H 40/67, A61B 5/145, A61B 5/369, A61B 5/389, A61B 5/398

(54) **IMPROVEMENTS RELATING TO SLEEP MONITORING**
VERBESSERUNGEN IM ZUSAMMENHANG MIT SCHLAFÜBERWACHUNG
PERFECTIONNEMENTS SE RAPPORTANT À LA SURVEILLANCE DU SOMMEIL

(30) Priority: 13.10.2017 AU 2017904161
(43) Date of publication of application: 19.08.2020
(73) Proprietor: Bioanalytics Holdings Pty Ltd, Melbourne, VIC 3004 (AU)
(72) Inventor: SASSE, Anthony, Melbourne, VIC 3004 (AU); JONES, Cyril, Melbourne, VIC 3004 (AU); RATTEN, Stephen, Melbourne, VIC 3004 (AU); MORGAN, Owen, Melbourne, VIC 3004 (AU)
(74) Representative: Bridle, Andrew Barry
(86) International application number: PCT/AU2018/000197
(87) International publication number: WO 2019/071291

(56) References cited:
- WO-A1-2015/187949
- WO-A1-2017/106840
- WO-A1-2017/218947
- WO-A2-2007/015833
- US-A1- 2008 035 157
- US-A1- 2012 123 225
- US-A1- 2013 131 464
- US-A1- 2014 114 165
- US-A1- 2015 073 232
- US-A1- 2017 020 391
- US-B1- 9 202 008

## Description

### Technical Field

This invention concerns a method and apparatus for use in monitoring the status of a person while they are asleep. In addition to its more general application, the invention is particularly adapted to use with an intraoral mandibular advancement device for treatment of obstructive sleep apnea.

### Background

There is a need for a low cost and convenient to use means by which the status of the body of a person can be monitored during sleep in order to record and/or provide an alert that a period of diminished breathing is underway.

Sleep apnea is a major concern for many people. The two major types of sleep apnea are Obstructive Sleep Apnea (OSA) and Central Sleep Apnea (CSA). In order to treat OSA, many different types of devices have been proposed, and are in use, for advancing the mandible of a person during sleep in order to provide a clearer airway. It has proven difficult to provide confirmation that such devices work and are efficacious in delivering the benefits claimed for them. An aim of the present invention is to provide means by which such confirmation may be achieved.

The principles of a particularly effective mandibular advancement device are described in international patent publication no. WO2006/072147. Some particularly preferred embodiments of the present invention preferably utilize a device of that general type. Another example of a mandibular advancement device is disclosed in international patent publication no. WO 2017/106840.

However, the present invention may otherwise be used, by way of other embodiments, in testing for, and monitoring of CSA where, rather than obstruction of the airway, the issue is a diminished stability in the body's feedback mechanisms that control respiration.

The present invention may also be used for monitoring the status of a person who, while not suffering from sleep apnea, may benefit from their condition being monitored while unwell or following surgery.

### Summary of Invention

Accordingly, in one aspect the invention provides a method of monitoring the status of a body of a person during sleep, according to claim 1. In an example useful for understanding the invention, a method of monitoring the status of a patient during sleep is provided, said method comprising:
- retaining in or adjacent the mouth or nose of said person an electronic detector which detects indications of the state of said body,
- wirelessly transmitting a first data stream representing said indications to a data receiving means,
- transmitting said first data stream from said data receiving means to a data processing means,
- processing said first data stream within said data processing means to produce a second data stream representing said indications, or conclusion drawn from said indications, and
- displaying a visual representation of said indications or conclusions.

Preferably said second data stream is transmitted wirelessly from said data processing means to a remote location. Preferably said wireless transmission of said first data stream occurs while said person is asleep and said data receiving means is adjacent said person.

Preferably for said person a single smartphone comprises:
- said data receiving means,
- said data processing means, and
- a means by which said transmission of the second data stream is made to said remote location.

The visual representation of said indications or conclusions may be displayed on said smartphone.

The remote location may comprise a computer facility which analyses corresponding said second data streams received from multiple smartphones.

Said electronic detector may be retained in position by a device bearing on the teeth and/or gingiva of said person. Said electronic detector may be retained by or within an intraoral mandibular advancement device being worn by said person.

Alternatively, said electronic detector may be mounted in a housing which is retained in position by adhesion or adhesive tape onto the face of the person.

Alternatively, said electronic detector may be mounted in a housing which is retained in position by a strap extending around the head of the person.

In another aspect the invention provides a mandibular advancement device according to claim 8. In an example useful for understanding the invention, a device for wearing by a person during sleep for the purpose of monitoring the status of said person's sleep is provided, said device comprising:
- a first portion adapted to be retained within the person's mouth,
- a second portion adapted to detect, from outside said body, indications of the state of said body, and
- means for wirelessly transmitting a first data stream representing said detected indications to a data receiving means located adjacent said sleeping person.

Preferably said data processing means is a smartphone.

According to the invention, the device comprises an intraoral mandibular advancement device adapted to be worn by said person.

Preferably said second portion adapted to detect indications of the state of said body comprises an electronic detector of airflow, audio and acceleration. It may also be adapted to detect indications of one or more of electro-musculogram data, electro-osculogram data, electro-encephalogram data and percent of saturated oxygen.

Said wireless transmission means is preferably a Bluetooth transmitter.

In another aspect the invention provides apparatus for monitoring the status of a body of a person during sleep, said apparatus comprising:
- a device according to claim 8, and
- a mobile smartphone including:
   - said data receiving means,
   - a data processing means for receiving said first data stream from said data receiving means, processing said first data stream to produce a second data stream representing said status, or an indication of said status, data, or conclusions drawn from said indications, and
   - means for transmitting from said data processing means to a remote location said second data stream representing said indications and/or an interpretation of, or conclusion drawn from, said indications.

Preferably said data processing means comprises software loaded into said smartphone.

### Brief Description of Drawings

In order that the invention may be more fully understood there will now be described, by way of example only, preferred embodiments and other elements of the invention with reference to the accompanying drawings where:
Figure 1 is a schematic of an apparatus and system according to a first embodiment of the invention;
Figure 2 is a schematic of an apparatus and system according to a second embodiment of the invention;
Figure 3 is a schematic of an apparatus and system according to a third embodiment of the invention;
Figure 4 is a perspective view of a mandibular advancement device forming part of the apparatus in Figure 1;
Figure 5 is a side view of the device in Figure 4;
Figure 6 is a plan view of the device in Figure 4;
Figure 7 is a frontal view of the device in Figure 4;
Figure 8 is a flowchart showing the operation of the software in an SCDA module in the system illustrated in Figure 3;
Figure 9 is a flowchart showing the operation of the software in a smartphone in the system illustrated in Figure 3; and
Figure 10 is a flowchart showing the operation of the software in a central data collection computer facility in the system illustrated in Figure 3.

With respect to the different embodiments of the invention, features which have equivalent function in each embodiment are identified by numerals which are one hundred different from the numerals identifying equivalent features in another embodiment.

### Description of Embodiments

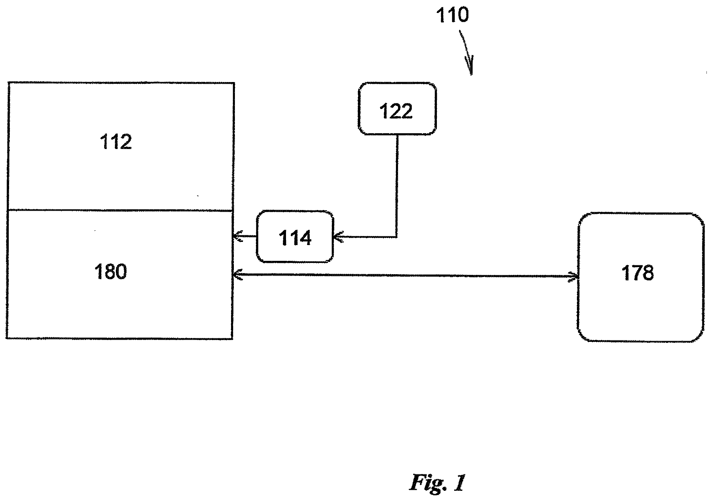

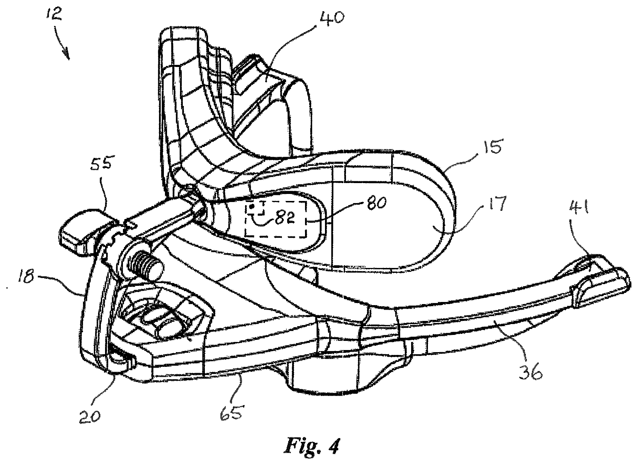

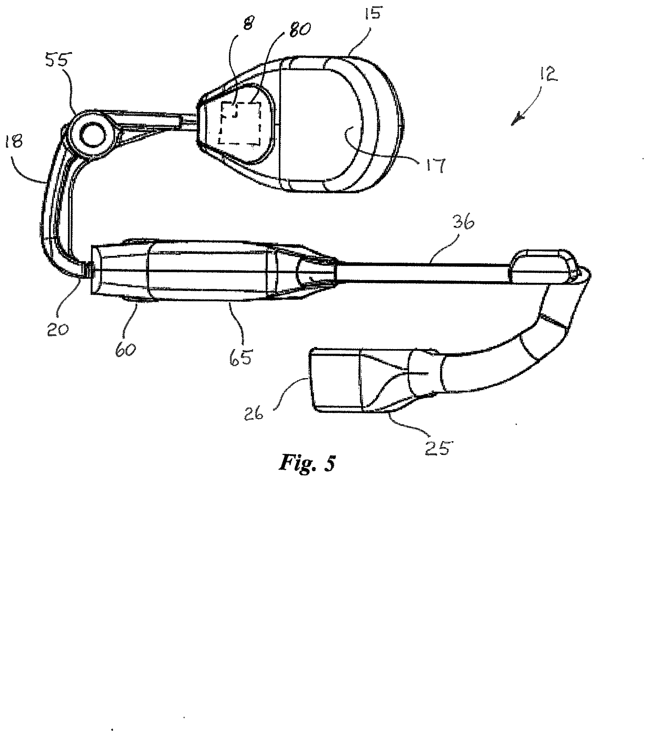

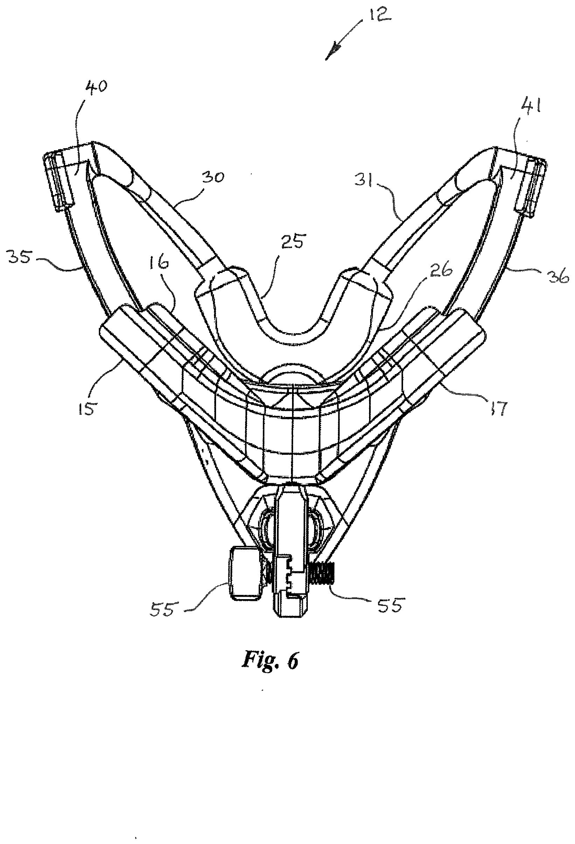

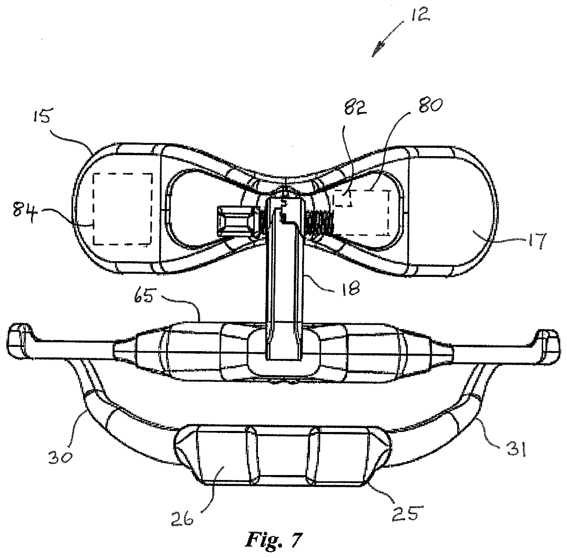

Referring to Figure 1, the first embodiment utilizes apparatus 110 incorporating a mandibular advancement device 112 the same as the device 12 shown in Figures 4 to 7.

As shown in Figures 4 to 7, the mandibular advancement device 12 incorporates a curved anterior maxillary abutment portion 15 mounted to an intraoral body portion 65 via an extraoral member portion 18. An arm 20 on the extraoral member 18 engages into the intraoral body portion 65. The effective length of the arm 20 is manually adjustable by the action of an adjustment wheel 60 whose central bore 61 is threadedly engaged with a thread 62 on the arm 20. The effective height of the extraoral member 18 can be adjusted by the angularly adjustable extraoral adjustment means 55 which adjusts the angle subtended by the articulation in the middle of the extraoral member 18.

The generally banana-shaped anterior mandibular abutment 25 is connected to the body portion 65 by connecting members 30 and 31. The intraoral posterior maxillary abutments 40 and 41 are connected to the intraoral body portion 65 by respective arms 35 and 36. In use the abutment surfaces 40 and 41 make contact with portion of the maxillary surface and that contact resists rotation of the device induced by the forces applied to the mandibular abutment surface 25 and the anterior maxillary abutment surface 15.

The concave surface 16 of the anterior maxillary abutment 15 is an elastomeric material that is shaped to fit comfortably upon the soft tissue and skin covering the user's sub-nasal maxillary bone. The anterior mandibular abutment 25 has a convex anterior face 26 which in use pushes on the user's gingiva covering their mandible. This arrangement allows the orthotic device to be applied to patients who have lost one or more of their anterior mandibular teeth. By abutting the gingiva covering the mandible, the anterior mandibular abutment surface 26 avoids or minimizes any undesirable movement of mandibular teeth over time.

An electronic module 80, specifically a sleep data collection and analysis module (hereinafter referred to as an SDCA module) is embedded within (or alternatively may be clipped over) the convex anterior face 17 of the maxillary abutment 15 at a region directly below the user's nose. A rechargeable electric battery 84 is also embedded within the concave face of the maxillary abutment 15 in order to provide an electric power source for the SDCA module 80. The battery 84, and much of the module 80, lie within cavities beneath the surface of the face 17 so their positions are indicated as dashed outlines.

The module 80 includes an electronic chip or chips on a printed circuit board (hereinafter referred to collectively as an SDCA detector system) 82 which acts as an electronic detector of the desired conditions. The device 12 is worn by the user during periods of sleep in order that relevant data may be collected by the SDCA detector system. The SDCA detector system includes two nasal and one oral sensors. The SDCA detector system is positioned so its exposed sensors are contacted by air passing into and out of the user's nose and/or mouth. The SDCA detector system detects acceleration of the user's upper lip, ambient audio and airflow past the sensors, stores that information, and transmits the data to a smartphone placed adjacent the user by means of a Bluetooth LE data link in near real time. An app loaded by the user into the smartphone processes the data and displays the results on the phone's screen.

Referring to Figure 1, concerning the first embodiment, when the user arises after sleep, they take off the mandibular advancement device 112 and place it on a base unit 114 which inductively charges the battery 84 after each night's sleep. The base unit 114 is powered from a USB charger 122. The base unit 114 is a docking station for the device 112 when the device 80 is not in use, and is configured to keep the device 112 clean and dust free. The base unit 114 has a region where relevant portions of the device 112 are soaked in an appropriate sterilizing fluid.

The user's smartphone 178 downloads the collected data from the SDCA module 80, which remains on the device 112. The SDCA module communicates with the smartphone 178 via a Bluetooth connection in order to upload the collected data from the SDCA module 80 while the user is wearing the device. The user's smartphone 178, placed near the sleeping user, is used to collect the data transmitted by the SDCA and to analyse the data to show the user the effectiveness of the device 112 for at least the previous sleep period or for other sleep periods as required. That analysis is performed by an app previously loaded by the user into the smartphone. The smartphone thus functions as a data processing means. There is accordingly a step of wirelessly transmitting a first data stream from the SDCA module 80 to a receiving means within the phone 178 after which, within the phone, the first data stream is transmitted to a data processing means where the data received is processed.

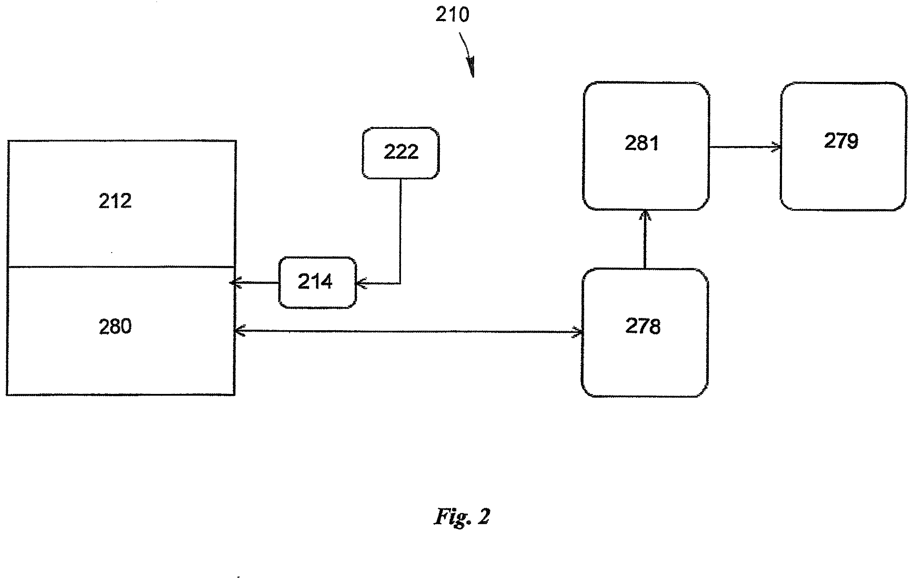

Referring to Figure 2, the apparatus 210 for the second embodiment is mostly the same as that described above for the first embodiment. A first main difference is that the SDCA module 280 has additional detection and recording functions when compared with the SDCA module 180 in Figure 1. The SDCA module 280 in Figure 2 records electro-musculogram (EMG) data, electro-osculogram (EOG) data, electro-encephalogram (EEG) data and percent of saturated oxygen (SA02) data in addition to the basic standard measurements of airflow, audio and acceleration recorded by the module 180 used in the first embodiment.

The second main difference is that the the smartphone 278 can upload the data to a suitable external repository 279 via the internet 281. Optionally the external repository 279 includes a central data analysis server which collects the data uploaded from multiple devices for appropriate health monitoring.

The electronics used in the base unit 214 are the same as the electronics used in the base unit 114.

By providing users with devices with different SDCA options, using different anterior maxillary abutment pads for the different SDCA options, the mandibular advancement device can be easily manufactured in a variety of versions. Pads for anterior maxillary abutment portions 15 can be easily interchanged. Even after purchase, a user can upgrade the product they have by purchasing an alternative version of the pad and thus change the mandibular advancement device to an enhanced version.

The invention provides higher level functions to a conventional mandibular advancement device which enhances its value and utility, so making it more attractive to users. The invention also provides confirmation of the effectiveness of the mandibular advancement device in assisting sleep behavior.

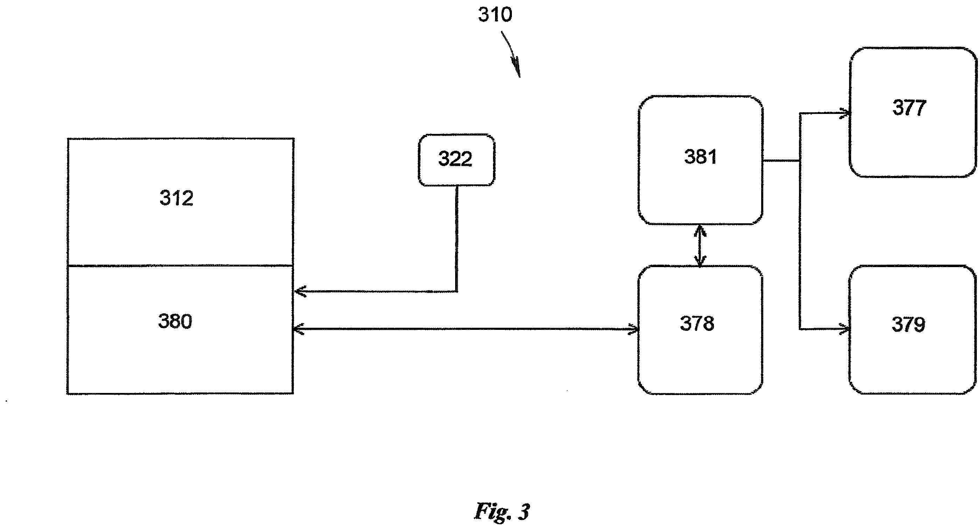

The apparatus 310 in the system illustrated by Figure 3 also provides for a local data collection on the user's home computer 377 as well as uploading to a global data collection database 379. The apparatus 310 also differs from the apparatus 210 (in Figure 2) in that the base unit 214 is not used. Instead the SDCA module 380 is charged directly from the charger 322.

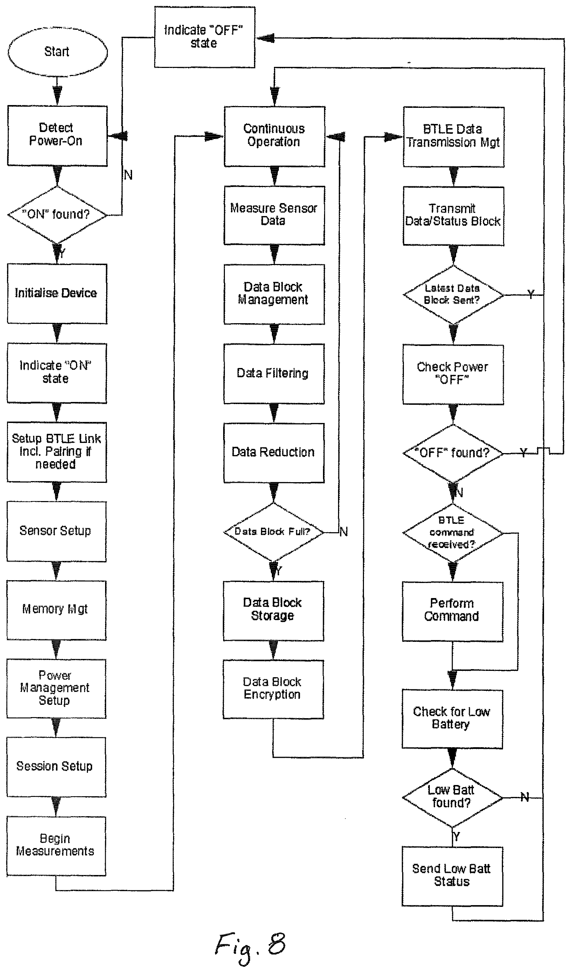

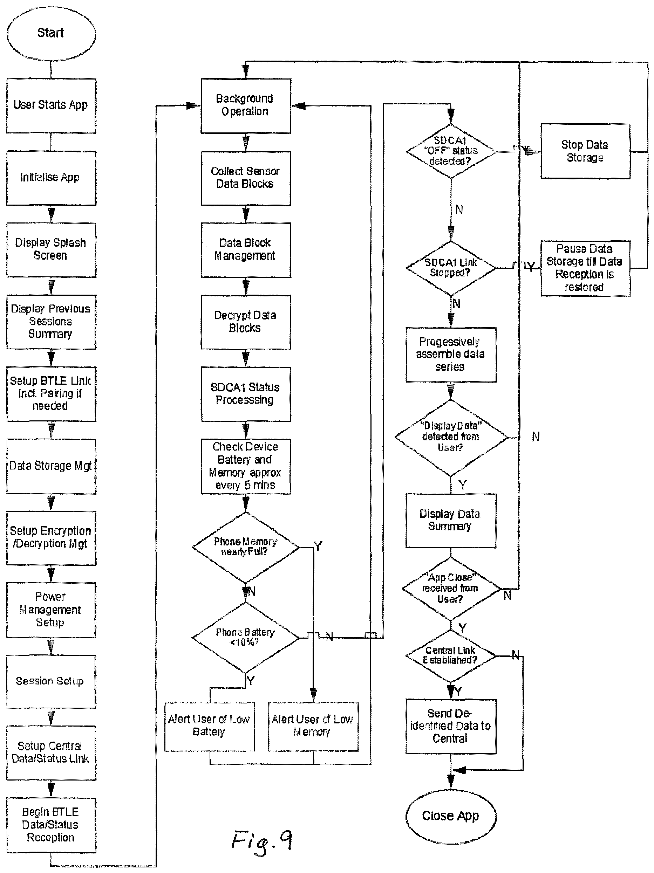

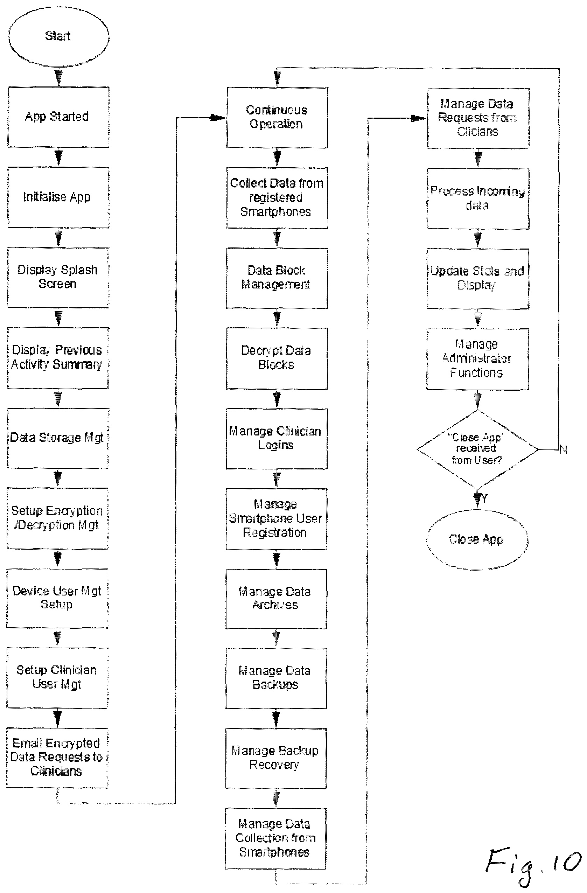

In relation to the operations indicated by, and generally self-explanatory from, the flowcharts in Figures 8 to 10, the following additional features are noted.
- All the data generated by the SDCA module 80 is stored in the module until it is able to be uploaded to the next component in the data collection/transmission chain.
- If the memory of the SDCA module 80, base module or smartphone are at capacity, the oldest data is discarded in favour of any new uploads of sleep data.
- The smartphone stores at least ten overnight data sets before needing to upload the data to the external repository 179.
- The smartphone stores all data sets uploaded from the SDCA until the smartphone memory is nearly exhausted.
- The user may elect to delete any or all data sets from the smartphone.
- The user is able to peruse a list of data sets and determine which they may wish to delete.
- The system utilizes data encryption between the SDCA module, the base unit and any attached smartphones.
- The system allows a clinician to log in remotely and view the the sleep behavior of a patient in near real time.

Alternative embodiments of the invention do not utilize a mandibular advancement device in order to retain the SDCA module and its battery power supply. Instead a component similar to the curved anterior maxillary abutment provides a housing and may be held beneath the user's nose by adhesive tape or by an elastic strap around the user's head. This could be used for sleep analysis of people who do not suffer from OSA and those who would be unacceptably disturbed by sleeping while a foreign object is in their mouth.

Whilst the above description includes the preferred embodiments of the invention, it is to be understood that many variations, alterations, modifications and/or additions may be introduced into the constructions and arrangements of parts previously described without departing from the scope of the invention, which is defined by the appended claims.

For example, the smartphone could be replaced by an alternative data processing means which has a suitable wireless data input such as Bluetooth LE. Its data output means (if present) may be wired or wireless.

It will be also understood that where the word "comprise", and variations such as "comprises" and "comprising", are used in this specification, unless the context requires otherwise such use is intended to imply the inclusion of a stated feature or features but is not to be taken as excluding the presence of other feature or features.

The reference to any prior art in this specification is not, and should not be taken as, an acknowledgment or any form of suggestion that such prior art forms part of the common general knowledge.

## Claims

1. A method of monitoring the status of a body of a person during sleep, said method comprising:
- retaining, within a mouth of said body, an intraoral body portion (65) of a mandibular advancement device (12), the mandibular advancement device further comprising an extraoral curved anterior maxillary abutment portion (15) mounted to the intraoral body portion via an extraoral member portion (18) and an electronic detector (82), being carried by the extraoral curved anterior maxillary abutment portion at a region directly below a nose of said body when the intraoral body portion is retained within said mouth, the extraoral member portion comprising:
a first arm portion (20) extending from a central bore (61) within the intraoral body portion, the first arm portion being threadedly engaged with a wheel (60) positioned in the central bore such that adjustment of the wheel is configured to facilitate adjustment of an effective length of extension of the first arm portion from the intraoral body portion, and
a second arm portion upon which the extraoral curved anterior maxillary abutment portion is mounted, the second arm coupled to the first arm portion via an articulation with an extraoral adjustment means (55) configured to adjust the angle subtended by the articulation, thereby adjusting the height of the extraoral member portion,
- detecting indications of a state of said body via the electronic detector, said indications including an airflow into and/or out of said nose, audio and acceleration data,
- wirelessly transmitting a first data stream representing said indications to a data receiving means,
- transmitting said first data stream from said data receiving means to a data processing means,
- processing said first data stream within said data processing means to produce a second data stream representing said indications, or conclusion drawn from said indications, and
- displaying a visual representation of said indications or conclusions.

2. The method according to claim 1 comprising wirelessly transmitting said second data stream from said data processing means to a remote location.

3. The method according to claim 1 wherein wirelessly transmitting said first data stream occurs while said person is asleep and said data receiving means is adjacent said person.

4. The method according to any one of claims 1 to 3 wherein for said person a single smartphone comprises:
- said data receiving means,
- said data processing means, and
- a means by which wirelessly transmitting the second data stream is made to said remote location.

5. The method according to claim 4 wherein said visual representation of said indications or conclusions is displayed on said smartphone.

6. The method according to claim 4 or 5 wherein said remote location comprises a computer facility which analyses corresponding said second data stream received from multiple smartphones.

7. The method according to any one of claims 1 to 6 wherein said electronic detector is retained in the region directly below the nose by the intraoral body portion bearing on the teeth and/or gingiva of said person.

8. A mandibular advancement device (12) adapted to be worn by a person during sleep for the purpose of monitoring a status of a body of said person during sleep, said device comprising:
- an intraoral body portion (65) adapted to be retained within a mouth of said body,
- an extraoral curved anterior maxillary abutment portion (15) mounted to the intraoral body portion via an extraoral member portion (18), the extraoral member portion comprising:
a first arm portion (20) extending from a central bore (61) within the intraoral body portion, the first arm portion being threadedly engaged with a wheel (60) positioned in the central bore such that adjustment of the wheel is configured to facilitate adjustment of an effective length of extension of the first arm portion from the intraoral body portion, and
a second arm portion upon which the extraoral curved anterior maxillary abutment portion is mounted, the second arm coupled to the first arm portion via an articulation with an extraoral adjustment means (55) configured to adjust the angle subtended by the articulation, thereby adjusting the height of the extraoral member portion,
- an electronic detector (82) being embedded within or clipped over a convex anterior face of the extraoral curved anterior maxillary abutment portion at a region directly below a nose of said body when the intraoral body portion is retained within said mouth and configured to detect, from outside said body, indications of the state of said body, said indications including an airflow into and/or out of said nose, audio and acceleration data, and
- means for wirelessly transmitting a first data stream representing said detected indications to a data receiving means located adjacent said sleeping person.

9. An apparatus for monitoring the status of a body of a person during sleep, said apparatus comprising:
- a mandibular advancement device according to claim 8, and
- a mobile smartphone (178, 278) including:
- said data receiving means,
- a data processing means for receiving said first data stream from said data receiving means, processing said first data stream to produce a second data stream representing said status, or an indication of said status, data, or conclusions drawn from said indications, and
- means for transmitting from said data processing means to a remote location said second data stream representing said indications and/or an interpretation of, or conclusion drawn from, said indications.

10. The apparatus according to claim 9 wherein said data processing means comprises software loaded into said smartphone.

## Patentansprüche

1. Verfahren zur Überwachung des Status eines Körpers einer Person während des Schlafs, wobei das Verfahren umfasst:
- Halten, innerhalb eines Munds des Körpers, eines intraoralen Körperabschnitts (65) einer mandibulären Protrusionsvorrichtung (12), die mandibuläre Protrusionsvorrichtung ferner umfassend einen extraoralen gekrümmten anterioren maxillären Widerlagerabschnitt (15), der an dem intraoralen Körperabschnitt über einen extraoralen Elementabschnitt (18) montiert ist, und einen elektronischen Detektor (82), der von dem extraoralen gekrümmten anterioren maxillären Widerlagerabschnitt in einem Bereich direkt unterhalb einer Nase des Körpers getragen wird, wenn der intraorale Körperabschnitt innerhalb des Munds gehalten wird, wobei der extraorale Elementabschnitt umfasst:
einen ersten Armabschnitt (20), der sich von einer zentralen Bohrung (61) innerhalb des intraoralen Körperabschnitts erstreckt, wobei der erste Armabschnitt in Gewindeeingriff mit einem Rad (60) steht, das in der zentralen Bohrung positioniert ist, sodass die Einstellung des Rads dazu konfiguriert ist, die Einstellung einer effektiven Länge der Verlängerung des ersten Armabschnitts von dem intraoralen Körperabschnitt zu ermöglichen, und
einen zweiten Armabschnitt, auf dem der extraorale gekrümmte anteriore maxilläre Widerlagerabschnitt montiert ist, wobei der zweite Arm über eine Artikulation mit einem extraoralen Einstellmittel (55) an den ersten Armabschnitt gekoppelt ist, das dazu konfiguriert ist, den von der Artikulation eingeschlossenen Winkel einzustellen und dadurch die Höhe des extraoralen Elementabschnitts einzustellen,
- Detektieren von Indikationen eines Zustands des Körpers über den elektronischen Detektor, wobei die Indikationen einen Luftstrom in die und/oder aus der Nase, Audio- und Beschleunigungsdaten beinhalten,
- drahtloses Übertragen eines ersten Datenstroms, der die Indikationen darstellt, an ein Datenempfangsmittel,
- Übertragen des ersten Datenstroms von dem Datenempfangsmittel an ein Datenverarbeitungsmittel,
- Verarbeiten des ersten Datenstroms innerhalb des Datenverarbeitungsmittels, um einen zweiten Datenstrom zu erzeugen, der die Indikationen oder eine aus den Indikationen gezogene Schlussfolgerung darstellt, und
- Anzeigen einer visuellen Darstellung der Indikationen oder Schlussfolgerungen.

2. Verfahren nach Anspruch 1, umfassend das drahtlose Übertragen des zweiten Datenstroms von dem Datenverarbeitungsmittel an einen Remote-Standort.

3. Verfahren nach Anspruch 1, wobei das drahtlose Übertragen des ersten Datenstroms erfolgt, während die Person schläft, und das Datenempfangsmittel neben der Person ist.

4. Verfahren nach einem der Ansprüche 1 bis 3, wobei, für die Person, ein einzelnes Smartphone umfasst:
- das Datenempfangsmittel,
- das Datenverarbeitungsmittel und
- ein Mittel, von dem aus das drahtlose Übertragen des zweiten Datenstroms an den Remote-Standort erfolgt.

5. Verfahren nach Anspruch 4, wobei die visuelle Darstellung der Indikationen oder Schlussfolgerungen auf dem Smartphone angezeigt wird.

6. Verfahren nach Anspruch 4 oder 5, wobei der Remote-Standort eine Computereinrichtung umfasst, die den entsprechenden zweiten Datenstrom analysiert, der aus mehreren Smartphones empfangen wird.

7. Verfahren nach einem der Ansprüche 1 bis 6, wobei der elektronische Detektor durch den intraoralen Körperabschnitt, der auf den Zähnen und/oder dem Zahnfleisch der Person aufliegt, in dem Bereich direkt unterhalb der Nase gehalten wird.

8. Mandibuläre Protrusionsvorrichtung (12), die dazu ausgelegt ist, von einer Person während des Schlafs, zwecks Überwachung eines Status eines Körpers der Person während des Schlafs, getragen zu werden, wobei die Vorrichtung umfasst:
- einen intraoralen Körperabschnitt (65), der dazu ausgelegt ist, innerhalb eines Munds des Körpers gehalten zu werden,
- einen extraoralen gekrümmten anterioren maxillären Widerlagerabschnitt (15), der an dem intraoralen Körperabschnitt über einen extraoralen Elementabschnitt (18) montiert ist, wobei der extraorale Elementabschnitt umfasst:
einen ersten Armabschnitt (20), der sich von einer zentralen Bohrung (61) innerhalb des intraoralen Körperabschnitts erstreckt, wobei der erste Armabschnitt in Gewindeeingriff mit einem Rad (60) steht, das in der zentralen Bohrung positioniert ist, sodass die Einstellung des Rads dazu konfiguriert ist, die Einstellung einer effektiven Länge der Verlängerung des ersten Armabschnitts von dem intraoralen Körperabschnitt zu ermöglichen, und
einen zweiten Armabschnitt, auf dem der extraorale gekrümmte anteriore maxilläre Widerlagerabschnitt montiert ist, wobei der zweite Arm über eine Artikulation mit einem extraoralen Einstellmittel (55) an den ersten Armabschnitt gekoppelt ist, das dazu konfiguriert ist, den von der Artikulation eingeschlossenen Winkel einzustellen und dadurch die Höhe des extraoralen Elementabschnitts einzustellen,
- einen elektronischen Detektor (82), der innerhalb einer konvexen anterioren Fläche des extraoralen gekrümmten anterioren maxillären Widerlagerabschnitts in einem Bereich direkt unterhalb einer Nase des Körpers eingebettet oder darüber geklipst ist, wenn der intraorale Körperabschnitt in dem Mund gehalten wird, und dazu konfiguriert ist, von außerhalb des Körpers Indikationen des Zustands des Körpers zu detektieren, wobei die Indikationen einen Luftstrom in die und/oder aus der Nase, Audio- und Beschleunigungsdaten beinhalten und
- Mittel zum drahtlosen Übertragen eines ersten Datenstroms, der die detektierten Indikationen darstellt, an ein Datenempfangsmittel, das sich neben der schlafenden Person befindet.

9. Gerät zur Überwachung des Status eines Körpers einer Person während des Schlafs, wobei das Gerät umfasst:
- eine mandibuläre Protrusionsvorrichtung nach Anspruch 8 und
- ein mobiles Smartphone (178, 278), beinhaltend:
- das Datenempfangsmittel,
- ein Datenverarbeitungsmittel zum Empfangen des ersten Datenstroms aus dem Datenempfangsmittel und Verarbeiten des ersten Datenstroms, um einen zweiten Datenstrom zu erzeugen, der den Status oder eine Indikation des Status, der Daten oder der aus den Indikationen gezogenen Schlussfolgerungen darstellt, und
- Mittel zum Übertragen, von dem Datenverarbeitungsmittel an einen Remote-Standort, des zweiten Datenstroms, der die Indikationen und/oder eine Interpretation der, oder Schlussfolgerung aus den, Indikationen darstellt.

10. Gerät nach Anspruch 9, wobei das Datenverarbeitungsmittel Software umfasst, die in das Smartphone geladen ist.

## Revendications

1. Procédé de surveillance de l'état du corps d'une personne pendant le sommeil, ledit procédé consistant à :
- retenir, à l'intérieur de la bouche dudit corps, une partie corps intrabuccal (65) d'un dispositif d'avancement mandibulaire (12), le dispositif d'avancement mandibulaire comprenant en outre une partie butée maxillaire antérieure incurvée extrabuccale (15) montée sur la partie corps intrabuccal par l'intermédiaire d'une partie élément extrabuccal (18), et un détecteur électronique (82) supporté par la partie butée maxillaire antérieure incurvée extrabuccale au niveau d'une région située directement au-dessous du nez dudit corps lorsque la partie corps intrabuccal est retenue à l'intérieur de ladite bouche, la partie élément extrabuccal comprenant :
une première partie bras (20) s'étendant depuis un alésage central (61) à l'intérieur de la partie corps intrabuccal, la première partie bras étant en prise par filetage avec une molette (60) positionnée dans l'alésage central de telle sorte qu'un réglage de la molette soit configuré pour faciliter le réglage d'une longueur utile d'extension de la première partie bras depuis la partie corps intrabuccal, et
une seconde partie bras sur laquelle la partie butée maxillaire antérieure incurvée extrabuccale est montée, le second bras étant accouplé à la première partie bras par l'intermédiaire d'une articulation avec un moyen de réglage extrabuccal (55) configuré pour régler l'angle sous-tendu par l'articulation, réglant ainsi la hauteur de la partie élément extrabuccal,
- détecter des indications d'un état dudit corps par l'intermédiaire du détecteur électronique, lesdites indications comprenant un flux d'air entrant dans ledit nez et/ou en sortant, des données audio et des données d'accélération,
- émettre sans fil un premier flux de données représentant lesdites indications vers un moyen de réception de données,
- émettre ledit premier flux de données depuis ledit moyen de réception de données vers un moyen de traitement de données,
- traiter ledit premier flux de données au sein dudit moyen de traitement de données pour produire un second flux de données représentant lesdites indications ou une conclusion tirée desdites indications, et
- afficher une représentation visuelle desdites indications ou conclusions.

2. Procédé selon la revendication 1, consistant à émettre sans fil ledit second flux de données depuis ledit moyen de traitement de données vers un emplacement distant.

3. Procédé selon la revendication 1, l'émission sans fil dudit premier flux de données se produisant pendant que ladite personne dort, et ledit moyen de réception de données étant adjacent à ladite personne.

4. Procédé selon l'une quelconque des revendications 1 à 3, pour ladite personne un unique smartphone comprenant :
- ledit moyen de réception de données,
- ledit moyen de traitement de données, et
- un moyen par lequel l'émission sans fil du second flux de données est effectuée vers ledit emplacement distant.

5. Procédé selon la revendication 4, ladite représentation visuelle desdites indications ou conclusions étant affichée sur ledit smartphone.

6. Procédé selon la revendication 4 ou 5, ledit emplacement distant comprenant une installation informatique qui analyse ledit second flux de données correspondant reçu depuis de multiples smartphones.

7. Procédé selon l'une quelconque des revendications 1 à 6, ledit détecteur électronique étant retenu dans la région située directement au-dessous du nez par la partie corps intrabuccal qui prend appui sur les dents et/ou la gencive de ladite personne.

8. Dispositif d'avancement mandibulaire (12) conçu pour être porté par une personne pendant le sommeil à des fins de surveillance d'un état du corps de ladite personne pendant le sommeil, ledit dispositif comprenant :
- une partie corps intrabuccal (65) conçue pour être retenue à l'intérieur de la bouche dudit corps,
- une partie butée maxillaire antérieure incurvée extrabuccale (15) montée sur la partie corps intrabuccal par l'intermédiaire d'une partie élément extrabuccal (18), la partie élément extrabuccal comprenant :
une première partie bras (20) s'étendant depuis un alésage central (61) à l'intérieur de la partie corps intrabuccal, la première partie bras étant en prise par filetage avec une molette (60) positionnée dans l'alésage central de telle sorte qu'un réglage de la molette soit configuré pour faciliter le réglage d'une longueur utile d'extension de la première partie bras depuis la partie corps intrabuccal, et
une seconde partie bras sur laquelle la partie butée maxillaire antérieure incurvée extrabuccale est montée, le second bras étant accouplé à la première partie bras par l'intermédiaire d'une articulation avec un moyen de réglage extrabuccal (55) configuré pour régler l'angle sous-tendu par l'articulation, réglant ainsi la hauteur de la partie élément extrabuccal,
- un détecteur électronique (82) noyé à l'intérieur d'une face antérieure convexe de la partie butée maxillaire antérieure incurvée extrabuccale, ou clipsé sur celle-ci, au niveau d'une région située directement au-dessous du nez dudit corps lorsque la partie corps intrabuccal est retenue à l'intérieur de la bouche, et configuré pour détecter, depuis l'extérieur dudit corps, des indications de l'état dudit corps, lesdites indications comprenant un flux d'air entrant dans ledit nez et/ou en sortant, des données audio et des données d'accélération, et
- un moyen permettant d'émettre sans fil un premier flux de données représentant lesdites indications détectées vers un moyen de réception de données situé de façon adjacente à ladite personne endormie.

9. Appareil de surveillance de l'état du corps d'une personne pendant le sommeil, ledit appareil comprenant :
- un dispositif d'avancement mandibulaire selon la revendication 8, et
- un smartphone mobile (178, 278) comprenant :
- ledit moyen de réception de données,
- un moyen de traitement de données permettant de recevoir ledit premier flux de données en provenance dudit moyen de réception de données, de traiter ledit premier flux de données afin de produire un second flux de données représentant ledit état, ou une indication dudit état, des données, ou des conclusions tirées desdites indications, et
- un moyen permettant d'émettre, depuis ledit moyen de traitement de données vers un emplacement distant, ledit second flux de données représentant lesdites indications et/ou une interprétation desdites indications ou bien une conclusion tirée de celles-ci.

10. Appareil selon la revendication 9, ledit moyen de traitement de données comprenant un logiciel chargé dans ledit smartphone.
